(19) Europäisches Patentamt
European Patent Office
Office européen des brevets

(11) Publication number: **0 194 794 A2**

# (12) EUROPEAN PATENT APPLICATION

(21) Application number: 86301506.1

(22) Date of filing: 04.03.86

(51) Int. Cl.[4]: A 61 K 37/64
//(A61K37/64, 31:70)

(30) Priority: 08.03.85 PC T/JP85/00118
30.04.85 PC T/JP85/00246

(43) Date of publication of application:
17.09.86 Bulletin 86/38

(84) Designated Contracting States:
BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Matsuo, Takao
1-74, Shimotsubayashi-rokutanda
Nishikyo-ku Kyoto 615(JP)

(72) Inventor: Horii, Satoshi
32-1, Miharadai 3-cho
Sakai Osaka 590-01(JP)

(72) Inventor: Kitamori, Nobuyuki
B-920, 45 Yamadaminami
Suita Osaka 565(JP)

(74) Representative: Laredo, Jack Joseph et al,
Elkington and Fife High Holborn House 52/54 High Holborn
London, WC1V 6SH(GB)

## (54) Saccharide digestion inhibiting composition.

(57) The present invention concerns a saccharide digestion inhibitor comprising a combination of an α-glucosidase inhibitor and non-pathogenic lactic acid producing live bacteria and a saccharide digestion inhibiting composition containing both of said components.

Oral administration of the saccharide digestion inhibitor or composition to man or animals results in the prevention or treatment of diabetes and obesity without resort to dieting and substantially without inducing side effects such as diarrhea, abdominal distension, etc. which are found with the α-glucosidase inhibiting substance administered alone.

EP 0 194 794 A2

Croydon Printing Company Ltd.

# Saccharide Digestion Inhibiting Composition

## Technical Field

The present invention relates to a saccharide digestion inhibitor comprising a combination of an α-glucosidase inhibitor and non-pathogenic lactic acid producing live bacteria and a saccharide digestion inhibiting composition containing them.

## Background Art

Recent years have seen an increasing number of obese people and diabetics due to overnutrition.

The following mechanism has been proposed for the genesis of obesity. Thus, as carbohydrates or saccharides in food are digested and absorbed as glucose, the blood glucose level rises, which triggers secretion of insulin from the pancreas. This insulin acts on the adipose tissue cells to cause uptake of blood glucose into the cells where it is converted to fats which in turn cause hypertrophy of the adipose cells, with the result that an abnormal accumulation of fats occurs in the body as

a whole to cause obesity.

On the other hand, diabetes is said to be caused by the following mechanism. Thus, when glucose is absorbed into the body, if insulin secretions are insufficient (Type I diabetes) or if insulin, despite its abundant supply, cannot display its function fully in the target tissue (Type II diabetes), there occurs hyperglycemia and, as a consequence, the glucose in an amount beyond a threshold level is excreted from the kidney into the urine to cause diabetes.

Therefore, administration of a drug which inhibits digestion of carbohydrates into glucose suppresses elevation of blood glucose level so that even diabetics with insufficient insulin secretion do not succumb to hyperglycemia. Inhibition of elevation of blood glucose leads to a decrease in insulin secretion and, hence, a decrease in synthesis of fat from glucose, thus serving to prevent or cure obesity. As the reduction of obesity potentiates the activity of insulin, it contributes to the prevention and treatment of diabetes due to the decreased activity of insulin. This line of thought prompted searches for inhibitors of carbohydrate digesting enzymes including α-glucosidase inhibitors and a variety of drugs having such activity have been developed.

However, when such a drug is administered to animals, large amounts of undigested oligosaccharides and disaccharides are retained in the intestinal tract and since they are not absorbed from the intestinal tract, there occurs a rise of osmotic pressure within the tract. The body of the host acts to lower the osmotic pressure to a normal physiological level by secreting a large amount of water into the intestinal tract across its wall, thus inducing osmotic diarrhea. Moreover, indigenous bacteria in the intestines utilize the retained oligosaccharides and disaccharides as energy sources and decompose them into carbon dioxide gas, hydrogen gas, methane gas, water and so on, which induce abdominal distention as well.

## Disclosure of the Invention

The present inventors conducted an intensive study to develop a method of inhibiting the diarrhea, abdominal distention and other adverse effects occurring after administration of the above α-glucosidase inhibitors and found that the object can be accomplished by administering such α-glucosidase inhibitors in combination with non-pathogenic lactic acid producing live bacteria.

Thus, as aforesaid, diarrhea, abdominal distention

and other adverse effects are caused by retention of large amounts of oligosaccharides and disaccharides in the intestinal tract but if lactic acid producing bacteria capable of utilizing and disposing of such saccharides and converting them to lactic acid are administered, the lactic acid produced thereby is quickly absorbed into the body to prevent onset of osmotic diarrhea. In this connection, if homo-lactic acid fermenting bacteria in particular are employed, lactic acid alone is produced without evolution of carbon dioxide gas, with the result that both diarrhea and abdominal distention can be prevented. On the other hand, the lactic acid produced is absorbed into the body from the intestinal tract wall but unlike absorption of glucose, it does not stimulate secretion of insulin so that there occurs no hypertrophy of adipose cells and the anti-obesity effect of the α-glucosidase inhibitor can be kept intact.

The α-glucosidase inhibitors employed in accordance with the present invention include, among others, the valiolamine derivatives described in European Patent Publication No. 56194 and No. 89812. These valiolamine derivatives may be

represented by the following general formula:

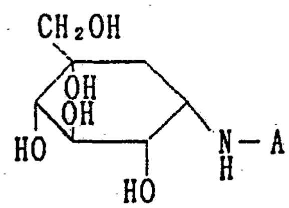

[I]

wherein A is an acyclic hydrocarbon group of 1 to 10 carbon atoms which may have one or more members of the group consisting of hydroxy, phenoxy, thienyl, furyl, pyridyl, cyclohexyl, and a substituted or unsubstituted phenyl group, a 5- to 6- membered cyclic hydrocarbon group which may have one or more members of the group consisting of hydroxy, hydroxymethyl, methyl and amino, or a saccharide residue.

Referring to the above general formula [I], A may for example be a straight-chain or branched, saturated or unsaturated, aliphatic hydrocarbon group of 1 to 10 carbon atoms, and the substituent or substituents on said substituted phenyl group may for example be lower alkyl, lower alkoxy, halogen, phenyl, etc. The saccharide residue means a group obtainable upon elimination of one hydrogen atom from a saccharide molecule, and may, for example, be a residue derived from a monosaccharide or oligosaccharide.

The following is a partial listing of the N-substituted valiolamine derivatives represented by

the general formula [I]:

(1) N-Phenethylvaliolamine

(2) N-(3-Phenylallyl)valiolamine

(3) N-furfurylvaliolamine

(4) N-Thenylvaliolamine

(5) N-(3-Pyridylmethyl)valiolamine

(6) N-(4-Bromobenzyl)valiolamine

(7) N-[(R)-β-Hydroxyphenethyl]valiolamine

(8) N-[(S)-β-Hydroxyphenethyl]valiolamine

(9) N-(β-Hydroxy-2-methoxyphenethyl)valiolamine

(10) N-(3,5-Di-tert-butyl-4-hydroxybenzyl)valiolamine

(11) N-(Cyclohexylmethyl)valiolamine

(12) N-Geranylvaliolamine

(13) N-(1,3-Dihydroxy-2-propyl)valiolamine

(14) N-(1,3-Dihydroxy-1-phenyl-2-propyl)valiolamine

(15) N-[(R)-α-(Hydroxymethyl)benzyl]valiolamine

(16) N-Cyclohexylvaliolamine

(17) N-(2-Hydroxycyclohexyl)valiolamine

(18) N-[(1R,2R)-2-Hydroxycyclohexyl]valiolamine

(19) N-(2-Hydroxycyclopentyl)valiolamine

(20) Methyl 4-[(1S,2S)-(2,4,5(OH)/3,5)-2,3,4,5-tetrahydroxy-5-(hydroxymethyl)cyclohexyl]amino-4,6-dideoxy-α-D-glucopyranoside

(21) Methyl 4-[(1S,2S)-(2,4,5(OH)/3,5)-2,3,4,5-tetrahydroxy-5-(hydroxymethyl)cyclohexyl]-amino-4-deoxy-α-D-glucopyranoside

(22) [(1S,2S)-(2,4,5(OH)/3,5)-2,3,4,5-Tetrahydroxy-5-(hydroxymethyl)cyclohexyl][(1R,2S)-(2,6/3,4)-4-amino-2,3-dihydroxy-6-(hydroxymethyl)cyclohexyl]-amine

(23) N-[(1R,2S)-(2,4/3,5)-2,3,4-Trihydroxy-5-hyhydroxy-methyl)cyclohexyl]valiolamine

(24) N-[(1R,2S)-(2,6/3,4)-4-Amino-2,3-dihydroxy-6-methylcyclohexyl]valiolamine

(25) N-[(1R,2S)-(2,6/3,4)-2,3,4-Trihydroxy-6-methyl-cyclohexyl]valiolamine

(26) N-[(1R,2S)-(2,4,6/3)-2,3,4-Trihydroxy-6-methylcyclohexyl]valiolamine

(27) 4-O-α-[4-[((1S)-(1,2,4,5(OH)/3,5)-2,3,4,5-Tetrahydroxy-5-(hydroxymethyl)cyclohexyl)amino]-4,6-dideoxy-D-glucopyranosyl]-D-glucopyranose

(28) 1,6-Anhydro-4-O-α-[4-[((1S)-(1,2,4,5(OH)/3,5)-2,3,4,5-tetrahydroxy —5— (hydroxymethyl)cyclohexyl)amino]-4,6-dideoxy-D-glucopyranosyl]-β-D-glucopyranose

Among the above derivatives, N-(1,3-dihydroxy-2-propyl)valiolamine, i.e. 1L(1S)-(1(OH), 2,4,5/1,3)-5-[[2-hydroxy-1-(hydroxymethyl)ethyl]amino]-1-C-(hydroxymethyl)-1,2,3,4-cyclohexanetetrol, is particularly suitable for the purposes of the present invention.

The following compounds may also be employed advantageously as the α-glucosidase inhibitor in

accordance with the present invention. Thus, N-substituted valienamine derivatives described in European Patent Publication No. 49981, for instance, which have the general formula:

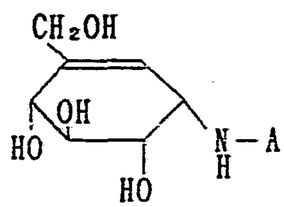

[II]

[wherein A is an acyclic hydrocarbon group of 1 to 10 carbon atoms which may have one or more members of the group consisting of hydroxy, phenoxy, thienyl, furyl, pyridyl, cyclohexyl and a substituted or unsubstituted phenyl, a 5- to 6-membered cyclic hydrocarbon group which may have one or more members of the group consisting of hydroxy, hydroxymethyl, methyl and amino, or a saccharide residue];

The N-substituted validamine derivatives described in European Patent Publication No. 56194, for instance, which have the general formula:

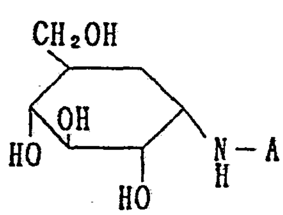

[III]

[wherein A is an acyclic hydrocarbon group of 1 to 10 carbon atoms which may be substituted by one or more members of the group consisting of hydroxy, phenoxy, thienyl, furyl, pyridyl, cyclohexyl and a substituted or unsubstituted phenyl, a 5- to 6-membered cyclic hydrocarbon group which may be substituted by one or more members of the group consisting of hydroxy, hydroxymethyl, methyl and amino, or a saccharide residue].

There may further be used acarbose (BAY g 5421; Naturwissenschaften, Vol. 64, pp. 535-537, 1977; Japanese Patent Publication No. 54-39474), trestatin (The Journal of Antibiotics, Vol. 36, pp. 1157-1175, 1983 and Vol. 37, pp. 182-186, 1984; Japanese Kokai Tokkyo Koho No. 54-163511), adiposins (The Journal of Antibiotics, Vol. 35, pp. 1234-1236, 1982); Denpun Kagaku (J. Jap. Soc. Starch Sci.), Vol. 26, pp. 134-144, 1979 and Vol. 27, pp. 107-113, 1980; Japanese Kokai Tokkyo Koho No. 54-106402; Japanese Kokai Tokkyo Koho No. 54-106403; Japanese Kokai Tokkyo Koho No. 55-64509; Japanese Kokai Tokkyo Koho No. 56-123986; Japanese Kokai Tokkyo Koho No. 56-125398), amylostatins (Agricultural and Biological Chemistry, Vol. 46, pp. 1941-1945, 1982; Japanese Kokai Tokkyo Koho No. 50-123891; Japanese Kokai Tokkyo Koho No. 55-71494; Japanese Kokai Tokkyo Koho No. 55-157595], oligostatins

(SF-1130X) Japanese Kokai Tokkyo Koho No. 53-26398; Japanese Kokai Tokkyo Koho No. 56-43294; The Journal of Antibiotics, Vol. 34, pp. 1424-1433, 1981), amino sugar compounds (Japanese Kokai Tokkyo Koho No. 54-92909), etc. The above compounds and other microorganism-derived α-glucosidase inhibitors are reviewed by E. Truscheit in Angewandte Chemie, Vol. 93, pp. 738-755, 1981.

Furthermore, there may also be used methyl 4-[(1S,6S)-(4,6/5)-4,5,6-trihydroxy-3-hydroxymethyl-2-cyclohexen-1-yl]amino-4,6-dideoxy-α-D-glucopyranoside obtained by methanolysis of acarbose and oligostatins C (182nd American Chemical Society National Meeting Abstracts of Papers MED I 69, August 1981, New York; The Journal of Antibiotics, Vol. 34, pp. 1429-1433, 1981; and Japanese Kokai Tokkyo Koho No. 57-24397), 1-deoxynojirimycin (Naturwissenschaften, Vol. 66, pp. 584-585, 1979) and N-substituted derivatives thereof, for example BAY m 1099 and BAY o 1248 [The Journal of Clinical Investigations, Vol. 14 (2-II), p. 47, 1984; Diabetologia, Vol. 27(2), 288A, 346A, 323A, 1984].

The non-pathogenic lactic acid producing live bacteria that can be used in accordance with the present invention are not limited only to lactic acid bacteria in the narrow sense of the term but also cover lactic

acid bacteria in the broad sense of the term such as live bacteria belonging to the genus Sporolabacillus and the genus Bifidobacterium which are enteric bacteria deficient of respiratory function. And those bacteria which do not give rise to gases which may cause abdominal distention in the process of growth and multiplication can be utilized with advantage.

As examples of the lactic acid producing bacteria that can be employed in the practice of the present invention include those of the genera Lactobacillus acidophilus, L. salivarius, L. delbrueckii, L. leichmannii, L. lactis, L. casei, Streptococcus faecalis, S. faecium, S. lactis, etc. Microorganisms of the genus Bifidobacterium produce 2 moles of lactic acid and 3 moles of acetic acid from 2 moles of glucose by fermentation, and Bifidobacterium bifidum, B. infantis, B. adolescentis, B. longum, etc. can be employed. However, these are only illustrative representative bacteria and the invention is not limited thereto. In a dry state, these microorganisms are vulnerable to air, oxygen, high temperature and humidity but the microorganisms of the genus Streptococcus are generally stable as compared with lactic acid producers of other genera and they are easy to handle.

And it is advantageous to employ indigenous enteric bacteria such as Streptococcus faecalis, Lactobacillus acidophilus, Bifidobacterium bifidum, etc. as the lactic acid producers, for these microorganisms will not disturb intestinal bacterial flora.

In administering an α-glucosidase inhibitor in combination with a non-pathogenic lactic acid producing strain in accordance with the present invention, the dosage of the α-glucosidase inhibitor is 0.05 to 500 mg/adult/dose, preferably 1.0 to 50 mg/adult/dose and this dose is preferably administered orally, usually twice to 4 times a day between one hour before a meal and 2 hours after a meal. Particularly when the α-glucosidase inhibitor is a valiolamine derivatives of the general formula [I], it is effective to administer the compound orally in doses of 1.0 to 20 mg/adult/dose 2 to 4 times a day, preferably at a suitable time between 1 hour before a meal and 2 hours after a meal.

On the other hand, as to the live non-pathogenic lactic acid bacteria to be used in combination with said inhibitors, it is effective to ingest a preparation containing $10^3$ to $10^{12}$ live cells/adult/dose, preferably $10^5$ to $10^{10}$ live cells/adult/dose, usually

2 to 4 times a day, preferably between one hour before a meal and 2 hours after a meal.

Regarding the method of administering the α-glucosidase inhibitor in combination with the non-pathogenic lactic acid producing live bacteria there may be mentioned the method in which a preparation containing α-glucosidase inhibitor and a preparation containing the lactic acid producing live bacteria are manufactured independently and the two preparations are administered simultaneously or at different times, and the method in which a preparation containing both the inhibitor and lactic acid bacteria is manufactured and administered, but it is in many cases expedient to provide a composition containing both the components.

With regard to the dosage forms of such preparations containing the α-glucosidase inhibitor and the lactic acid producing bacteria respectively or of the compound preparation containing the two components together, one may use the dosage forms convenient to administer in view of the physical chemical and biological properties of the respective components, such as, for example, tablets, pellets, fine granules, granules, powders, capsules, solutions and suspensions. Furthermore, each of such preparations may contain

suitable additional components selected from among the excipients, colors, flavors, stabilizers, gloss improving agents, etc. which are routinely used in pharmaceutical practice.

The saccharide digestion inhibiting composition containing said α-glucosidase inhibitor and non-pathogenic lactic acid producing bacteria can be manufactured by mixing the two components by a known method of pharmaceutical manufacture. The proportions of α-glucosidase inhibitor and non-pathogenic lactic acid producing bacteria in the composition is preferably such that $10^3$ to $10^{12}$ cells of the latter are available to 0.05 to 500 mg of the former, preferably $10^5$ to $10^{10}$ cells of the latter to 1.0 to 50 mg of the former.

As lactic acid producing live bacteria are generally anaerobic and sensitive to air or oxygen in a dry state and, also vulnerable to high temperature and humidity, they are preferably handled in the presence of an inert gas or in vacuo and at low temperature.

To provide the composition according to the present invention in the form of a solid preparation, the powders may simply be admixed or the powders may be compressed into granules or tablets. To manufacture such granules or tablets by the wet method, they can be

mixed using an aqueous binder solution and the mixture dried to give the desired solid preparation. Furthermore, the powders or granules obtained as above can be filled into capsules to provide an encapsulated preparation.

In order to mix small amounts of the active components with large amounts of other powders to provide a homogeneous mixture, the so-called stepwise mixing method can be advantageously adopted. For example, the active components are mixed with 100 to 200 volumes of powders to provide a homogeneous powder which is then mixed with the remainder of powders to provide a uniform powder.

Drying from an aqueous composition can be effected by such means as L-drying, freeze-drying, spray drying, etc.

To obtain dry cells of lactic acid producing bacteria, it is preferable to suspend wet cells in a neutral buffer containing a suitable stabilizer such as monosodium glutamate, adonitol or the like and dry the suspension by a method known per se.

The composition according to the present invention is effective in the prevention or treatment of obesity and diabetes at low doses, can be administered easily and produces little side effects.

Further, what is generally called diarrhea occurs as the result of indigestion, abnormal fermentation in the intestines or a disturbance of the intestinal bacterial flora, and, according to the causes, antibiotics, Chinese formula medicines, lactic acid bacteria, medicinal charcoal, etc. are employed.

Thus, antibiotics, creosote and other compounds are used for obtaining bactericidal or bacteriostatic effects on intestinal bacteria which cause diarrhea. As to Chinese formula medicines, while their actions are not necessarily well established, it is said that the astringent action due to their main principle tannin is the chief efficacy.

Lactic acid bacteria are claimed to be of use for normalizing the disturbed balance of intestinal bacterial flora due to diarrhea or for the treatment of indigestion but its mode of action remains yet to be fully known.

Medicinal charcoal has been used for adsorption of gases produced by abnormal fermentation or adsorption and detoxication of toxic substances in autointoxication.

As mentioned hereinbefore, the lactic acid producing bacteria incorporated in the composition of the present invention selectively utilize the saccharides detained in the intestinal tract on account of the inhibition of

carbohydrate digestion by the other component α-glucosidase inhibitor , and thereby, diarrhea and abdominal distention are prevented. Moreover, said lactic acid producing bacteria are indigenous bacteria in the intestines and are harmless and the lactic acid produced on their growth is also quite harmless to humans. Therefore, the composition according to this invention can be said to be quite safe.

Example 1

To 88 g of potato starch were added $10^{12}$ live cells of Bifidobacterium bifidum (IFO-14252) (10 g as cell powder) and 1 g of powdery N-(1,3-dihydroxy-2-propyl)valiolamine (hereinafter referred to as AO-128) and the mixture was milled in a mortar for 10 minutes. Then, 1 g of magnesium stearate powder was added and the mixture was further milled thoroughly for 3 minutes to provide a powdery composition.

Example 2

To 59 g of lactose and 25 g of potato starch were added $10^{12}$ live cells of Bifidobacterium bifidum (IFO-14252) (10 g as cell powder) and 5 g of AO-128 powder and the mixture was thoroughly milled in the mortar for 10 minutes. Then, 1 g of magnesium stearate was added and the

mixture was further milled for 3 minutes to provide a powdery composition. Using 100 mg portions of this powdery composition, flat tablets with a diameter of 6.5 mm were manufactured at the compressive pressure of 500 $kg/cm^2$. Six beagle dogs (body weight: 9.1-10.7 kg) were each dosed orally with 1 tablet immediately before feeding and observed for 48 hours after administration. None of the dogs developed diarrhea.

On the other hand, using 59 g of lactose and 35 g of potato starch, 5 g of AO-128 and 1 g of magnesium stearate, the above procedure was followed to prepare compression tablets (hereinafter referred to as AO-128 tablets) containing 5 mg of AO-1285 in 100 mg per tablet.

Further, 59 g of lactose and 30 g of potato starch, $10^{12}$ live cells of Bifidobacterium bifidum (IFO-14252) (10 g as cell powder) and 1 g of magnesium stearate were treated in the same manner as above to prepare tablets (hereinafter referred to as bacterial tablets) containing $10^9$ cells in 100 mg per tablet.

One AO-128 tablet was administered orally to each of 3 beagle dogs 30 minutes before feeding and, then, one bacterial tablet was further administered orally immediately before feeding. The animals were observed for 24 hours. None of the dogs developed diarrhea.

In contrast, when the AO-128 tablet alone was orally administered to each of 3 beagle dogs 30 minutes before feeding and the animals were observed without giving the bacterial tablet, all the dogs showed abdominal distention and diarrhea over the period of 5 to 8 hours after feeding.

Example 3

In a mortar, 20 g of lactose, 54 g of corn starch, $10^{12}$ live cells of Lactobacillus acidophilus (IFO-13951) (10 g as cell powder), $10^{12}$ live cells of Streptococcus faecalis (IFO-12580) (10 g as cell powder) and 5 g of AO-128 powder were thoroughly milled for 10 minutes. Then, 1 g of magnesium stearate was added and the mixture was further stirred for 3 minutes. Using 100 mg portions of the mixture, flat tablets with a diameter of 6.5 mm were molded at the compressive pressure of 500 $kg/cm^2$. One of the above tablets was orally administered to each of 6 beagle dogs immediately before feeding and the animals were observed for 48 hours after administration. None of the dogs developed diarrhea. When the same tablet was orally administered to each of 3 healthy males immediately before lunch and the subjects were observed for 10 hours, none of them developed diarrhea although mild abdominal distention was observed.

On the other hand, 20 g of lactose, 74 g of corn starch, 5 g of AO-128 powder and 1 g of magnesium stearate were treated in the same manner as above to prepare tablets free of lactic acid producing bacteria (containing 5 mg of AO-128 in 100 mg per tablet). One tablet was administered to each of the same subjects under the same conditions. Abdominal distension and diarrhea were found over the period of 5 to 8 hours after administration.

Example 4

A powder prepared by mixing lactose and corn starch in a weight ratio of 8 to 2, AO-128 powder, hydroxypropylcellulose and distilled water were processed by the wet method to prepare granules (hereinafter referred to as AO-128 granules) containing 5 mg of AO-128 and 3 mg of hydroxypropylcellulose in 100 mg. One hundred grams of the granules were thoroughly mixed with $9.5 \times 10^{11}$ live cells of Bifidobacterium bifidum (IFO-14252) (9.5 g as cell powder) and the mixture was molded with a tableting machine to prepare flat tablets weighing 110 mg and with a diameter of 6.5 mm. One of these tablets was orally administered to each of 3 beagle dogs immediately before feeding and the animals were observed for 48 hours. None of the dogs developed diarrhea.

On the other hand, 100 g of AO-128 granules were mixed with 1 g of magnesium stearate and flat tablets weighing 101 mg and with a diameter of 6.5 mm were prepared in the same manner as above. One of the tablets was orally administered to each of 3 beagle dogs immediately before feeding. Though varying in severity, diarrhea was found in all animals during the period of 4 to 10 hours after administration.

Example 5

The same mixed powder as used in Example 4, AO-128, Streptococcus faecalis (IFO-12580) and polyvinyl pyrrolidone were processed by the wet method to provide granules containing 10 mg of AO-128, $10^{10}$ cells and 30 mg of polyvinyl pyrrolidone in 1 gram.

Reference Example 1

One gram of the mixture prepared in Example 1 and one gram of cells of lactic acid producing bacteria of a kind different from the bacteria used in Example 1 were mixed with 99 g of commercial powder feed (CE-2) to give a test ration. This ration was given to male Jcl:SD rats aged 7 weeks (6 animals per group) and the property of feces excreted after 36 to 48 hours was examined. Control animals received a mixture of 10 mg of AO-128 and 100 g of powder feed. The results are shown in Table 1.

Table 1

| Organism | Property of feces* | | |
| --- | --- | --- | --- |
| | + | ± | − |
| Lactobacillus acidophilus (IFO-13951) | 1 | 2 | 3 |
| Streptococcus faecalis (IFO-12580) | 1 | 1 | 4 |
| Bifidobacterium bifidum (IFO-14252) | 0 | 3 | 3 |
| None (Control group) | 6 | 0 | 0 |

* +: Feces unformed (watery stool).

±: Fecal masses, a diffusion of water noted on filter paper.

−: Fecal masses, no diffusion of water on filter paper.

## Reference Example 2

Using 2 or more different bacteria, test rations were prepared by the same procedure as Reference Example 1. However, the amount of potato starch was reduced to adjust for the volume increase due to the cells to provide a composition containing cells of AO-128 and the composition was mixed with feed. The resulting ration was given to 7-week-old Jcl:SD rats (5 animals per group) and the property of feces after 36 to 48 hours was observed. Control rats received a mixture of 10 mg of AO-128 and 100 g of powder feed. The results are shown in Table 2.

Table 2

| Organisms | Lactobacillus acidophilus (A)<br>Streptococcus faecalis (F)<br>Bifidobacterium bifidum (B) | Property of feces | | |
|---|---|---|---|---|
| | | + | ± | - |
| | A + B | 0 | 3 | 2 |
| | A + F | 0 | 2 | 3 |
| | B + F | 0 | 0 | 5 |
| | A + B + F | 0 | 1 | 4 |
| | None (Control group) | 3 | 2 | 0 |

It is apparent that the diarrhea due to AO-128 can be remarkably inhibited by the administration of lactic acid bacteria, whether of one and the same kind or of two or more kinds.

Reference Example 3

Though the composition of the present invention mitigates diarrhea, the possibility could not be ruled out that it might induce changes in the effects on hyperglycemia and hyperinsulinemia following a diet intake and in anti-obesity effect. Therefore, these aspects were investigated.

Influence on dietary hyperglycemia and hyperinsuliemia

The rations prepared in the same manner as

Reference Example 1 were given to 8-week-old male Jcl:SD rats (6 individuals per group) which had not been fed for 24 hours to investigate how the administration of lactic acid producing bacteria would influence the inhibitory effect of AO-128 on the hyperglycemia and hyperinsuleinemia following a diet intake.

Blood samples were collected from the tail vein immediately before feeding and 30, 60 and 120 minutes after feeding and the plasma glucose and insulin levels were determined. The result are set forth in Tables 3 and 4.

Table 3

Plasma glucose level

| Group | AO-128 (%) | Lactobacillus asidophilus, Streptococcus faecalis, Bifidobacterium bifidum (Number of cell/g) | Plasma glucose level mg/dl 0 min. | 30 min. | 60 min. | 120 min. |
|---|---|---|---|---|---|---|
| 1 | 0 | 0 | 101 ± 6 | 179 ± 13 | 184 ± 6 | 189 ± 11 |
| 2 | 0 | 10" each | 98 ± 10 | 186 ± 13 | 182 ± 13 | 178 ± 11 |
| 3 | 0.01 | 0 | 101 ± 10 | 141 ± 8[a] | 154 ± 11[a] | 152 ± 11[a] |
| 4 | 0.01 | 10" each | 96 ± 2 | 146 ± 10[a] | 155 ± 10[a] | 147 ± 8[a] |

Mean ± Standard deviation (n = 6)

Significant difference from Group 1 (a:P < 0.001, b:P < 0.01)

Table 4

Plasma insulin level

| Group | AO-128 (%) | Lactobacillus asidophilus, Streptococcus faecalis, Bifidobacterium bifidum (Number of cell/g) | Plasma insulin level mg/dl | | | |
|---|---|---|---|---|---|---|
| | | | 0 min. | 30 min. | 60 min. | 120 min. |
| 1 | 0 | 0 | 32± 6 | 80±14 | 70±16 | 60±22 |
| 2 | 0 | $10^8$ each | 35± 5 | 85±18 | 72±12 | 58±13 |
| 3 | 0.01 | 0 | 34± 8 | 52± 6[a] | 56±10 | 48±10 |
| 4 | 0.01 | $10^8$ each | 31± 9 | 56±11[a] | 61±12 | 50±16 |

Mean ± Standard deviation ($n=6$)

Significant difference from Group 1 (a:$P<0.001$, b:$P<0.01$)

As the results indicate, the inhibitory effect of AO-128 on dietary hyperglycemia and hyperinsulinemia was not affected at all by the combined use of bacterial cells.

Reference Example 4

The rations prepared by the same procedure as Reference Example 1 were fed to 3-week-old male rats with Zucker obesity for 28 days to investigate changes in body weight gain, food consumption, feed conversion and body fat. The result are shown in Table 5.

Table 5

| Test group | 1 | 2 | 3 |
|---|---|---|---|
| A O − 128 | 0 | 0.01% | 0.01% |
| Lactobacillus acidophilus | 0 | $10^8$/g | 0 |
| Streptococcus faecalis | 0 | $10^8$/g | 0 |
| Bifidobacterium bifidum | 0 | $10^8$/g | 0 |
| body weight gain(g/28day) | 214±10 | $144 \pm 22^a$ | $132 \pm 28^a$ |
| food consumption(g/28day) | 772±52 | $579 \pm 62^a$ | $602 \pm 56^a$ |
| food conversion (%) | 27.7±0.8 | $24.8 \pm 1.4^b$ | $21.9 \pm 3.6^b$ |
| body fat (%) | 27.1±2.6 | $21.4 \pm 3.2^b$ | $19.8 \pm 5.4^b$ |
| diarrhea | 0/6 | 1/6 | 6/6 |

Mean±Standard deviation(n= 6) Significant difference from Group I (a: $P < 0.001$, b: $P < 0.01$, c: $P < 0.02$)

In the analysis for body fat, the liver, the adipose tissue of the epididymis, the digestive tract and its contents were removed and the remainder was used as a sample. The rations containing the bacterial cells were prepared every other day and fed. Between groups 2 and 3, no significant difference was found in any of the parameters investigated.

Reference Example 5

Male Wistar Kyoto rats aged 1.5 days (N-STZ-1.5) or 5 days (N-STZ-5) were subcutaneously dosed with a solution of Streptozotocin (120 mg/kg body weight) in 0.1 M citrate buffer to construct diabetic models. Control rats were subcutaneously treated with 0.1 M citrate buffer at the age of 1.5 days. When these rats reached the age of 9 weeks, a ration containing $10^8$ cells each of Lactobacillus acidophilus, Bifidobacterium bifidum and Streptococcus faecalis per gram or a ration prepared by adding 0.01% of AO-128 to the above ration and the animals in both groups were reared for 14 days. A blood sample was taken from the tail vein and the plasma glucose levels was determined. Each animal was then housed in a metabolic cage for 2 days and the food consumption, water intake, urine volume and urine sugar excretion were measured. The rations were prepared

freshly every other day and fed. The results are shown in Table 6.

Table 6

| Rat | Group | Plasma glucose (mg/dl) | Food consumption (g/day) | Water intake (ml/day) | Urine volume (ml/day) | Urine sugar excretion (g/day) |
|---|---|---|---|---|---|---|
| Normal | Test group | 126 ± 3 | 17.7 ± 2.7 | 38.9 ± 5.5 | 13.8 ± 2.3 | 0 |
| | Control group | 117 ± 5[b] | 15.8 ± 1.6 | 45.1 ± 6.9 | 15.7 ± 1.1 | 0 |
| N-STZ-1.5 | Test group | 161 ± 19 | 15.3 ± 1.1 | 38.1 ± 5.0 | 14.0 ± 2.3 | 0.04 ± 0.09 |
| | Control group | 138 ± 8[b] | 15.1 ± 1.9 | 45.8 ± 6.6 | 15.7 ± 5.6 | 0 |
| N-STZ-5 | Test group | 453 ± 18 | 27.1 ± 2.0 | 151 ± 27 | 121 ± 31 | 6.76 ± 2.60 |
| | Control group | 319 ± 80[a] | 18.2 ± 2.2[a] | 58.8 ± 11.3[a] | 21.7 ± 8.3[a] | 0.49 ± 0.37[a] |

Mean ± Standard deviation ($n = 5 \sim 7$). Significant difference from each control group (a: $P < 0.001$, b: $P < 0.01$).

As the results indicate, the combination of AO-128 and lactic acid producing bacteria caused a significant suppression of plasma glucose in rats with mild diabetes.

Reference Example 6

Acute toxicity study

In an acute toxicity study in rats, oral administration of AO-128 at the level of 10 g/kg body weight caused no death. When this was mixed with $10^{11}$ cells of any of the 3 different lactic acid producing bacteria used in the present invention and the mixture was orally administered at the same time as AO-128 (10 g/kg body weight), no influence was observed.

Industrial Applicability

The saccharide digestion inhibitor and the saccharide digestion inhibitory composition, both according to the present invention, are useful prophylactic and therapeutic agents for diabetes and obesity in man and animals, for oral administration of either of them prevents and cures diabetes and obesity without requiring dieting and substantially without causing diarrhea and other side effects.

What is claimed is:

1. A saccharide digestion inhibitor comprising a combination of an α-glucosidase inhibitor and non-pathogenic lactic acid producing live bacteria.

2. A saccharide digestion inhibitor according to Claim 1 wherein said α-glucosidase inhibitor is a valiolamine derivative of the general formula:

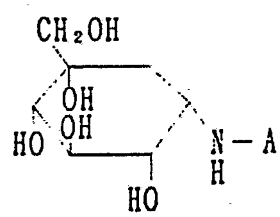

wherein A is an acyclic hydrocarbon group of 1 to 10 carbon atoms which may have one or more members selected from the group consisting of hydroxy, phenoxy, thienyl, furyl, pyridyl, cyclohexyl, and a substituted or unsubstituted phenyl; a five- to six-membered cyclic hydrocarbon group which may have one or more members selected from the group consisting of hydroxy, hydroxymethyl, methyl and amino, or a saccharide residue.

3. A saccharide digestion inhibitor according to Claim 1 wherein said α-glucosidase inhibitor is N-(1,3-dihydroxy-2-propyl)valiolamine.

4. A saccharide digestion inhibitor according to Claim 1 wherein the ratio of combination of said α-glucosidase inhibitor to said non-pathogenic lactic acid producing live bacteria is 0.1-400 mg to $10^3$-$10^{12}$ cells.

5. A saccharide digestion inhibiting composition containing an α-glucosidase inhibitor and non-pathogenic

lactic acid producing live bacteria.

6. A composition according to Claim 5 wherein said α-glucosidase inhibitor is a valiolamine derivative of the general formula:

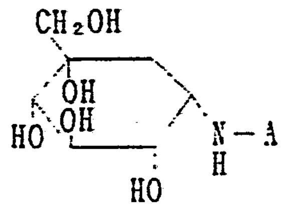

wherein A is an acyclic hydrocarbon group of 1 to 10 carbon atoms which may have one or more members selected from the group consisting of hydroxy, phenoxy, thienyl, furyl, pyridyl, cyclohexyl, and a substituted or unsubstituted phenyl; a five- to six-membered cyclic hydrocarbon group which may have one or more members selected from the group consisting of hydroxy, hydroxymethyl, methyl and amino, or a saccharide residue.

7. A composition according to Claim 5 wherein said α-glucosidase inhibitor is N-(1,3-dihydroxy-2-propyl)valiolamine.

8. A composition according to Claim 5 wherein the ratio of the α-glucosidase inhibitor to the non-pathogenic lactic acid producing live bacteria is 0.1-400 mg to $10^3$-$10^{12}$ cells.

9. The use of a saccharide digestion inhibitor according to claim 1 or a saccharide digestion inhibitor composition according to claim 5 for the manufacture of a medicament for the prevention or treatment of diabetes and obesity.